(19) Europäisches Patentamt
European Patent Office
Office européen des brevets

(11) Veröffentlichungsnummer : **0 022 062**
**B1**

# (12) EUROPÄISCHE PATENTSCHRIFT

(45) Veröffentlichungstag der Patentschrift : **30.03.83**

(51) Int. Cl.³ : **C 09 B 3/04**, C 07 C 50/18, C 25 B 3/04

(21) Anmeldenummer : **80810174.5**

(22) Anmeldetag : **27.05.80**

## (54) Elektrochemisches Verfahren zur Herstellung von Benzanthron.

(30) Priorität : **30.05.79 CH 5034/79**

(43) Veröffentlichungstag der Anmeldung : **07.01.81 Patentblatt 81/01**

(45) Bekanntmachung des Hinweises auf die Patenterteilung : **30.03.83 Patentblatt 83/13**

(84) Benannte Vertragsstaaten : **BE CH DE FR GB LI**

(56) Entgegenhaltungen :
**DE C 585 428**
**US A 787 859**
**N.N. WOROSHZOW « Grundlagen der Synthese von Zwischenprodukten und Farbstoffen, AKADEMIE-VERLAG, Berlin, 1966 Seiten 833-838, 847, 848, 907-910, 964, 965**

(73) Patentinhaber : **CIBA-GEIGY AG**
**Patentabteilung Postfach**
**CH-4002 Basel (CH)**

(72) Erfinder : **Bersier, Jacques, Dr.**
**Gstaltenrainweg 61**
**CH-4125 Riehen (CH)**
Erfinder : **Jäger, Horst, Dr.**
**Wyhlenweg 10**
**CH-4126 Bettingen (CH)**
Erfinder : **Schwander, Hansrudolf, Dr.**
**Unterm Schellenberg 189**
**CH-4125 Riehen (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 022 062 B1

Elektrochemisches Verfahren zur Herstellung von Benzanthron

Gegenstand der Erfindung ist ein elektrochemisches Verfahren zur Herstellung von Benzanthron ausgehend von Anthrachinon und dessen Reaktion mit Glyzerin, dadurch gekennzeichnet, dass man Anthrachinon, das noch elektronegative Substituenten, wie z. B. $OH^-$ bzw. Halogen enthalten kann, in einer Elektrolysezelle in saurem Medium kathodisch reduziert und die reduzierte Semichinonform gleichzeitig mit dem Glyzerin unter Ringschluss zum entsprechenden Benzanthron reagiert.

Die Herstellung entsprechender Benzanthrone auf anderem Wege ist bekannt, jedoch wurdenstets Reduktionssysteme benötigt, bei denen vorwiegend Metalle als Reduktionsmittel zum Einsatz kamen.

Schon Bally B. *38* (1905), beschreibt z. B. die Herstellung von Benzanthron aus Anthrachinon und Glyzerin und einem Reduktionsmittel.

Seither wurde verschiedentlich versucht diese konventionelle Ringschlusmethode zu verbessern.

Beispielsweise erlangte Anilin als Reduktionsmittel [Scholl und Bally, B. *44*, 1956 (1911)], keine wirtschaftliche Bedeutung. Die Verwendung von z. B. 70- bis 90 %iger Schwefelsäure als Reaktionsmedium und Metallen, wie Eisen, Zink, Aluminium oder Kupfer, als Reduktionsmittel sind in folgenden Schriften beschrieben : US-Patent 1.896.147 (Reduktionsmittel Fe). US-Patent 2.034.485 und UdSSR-Patent 401-130 (Reduktionsmittel Fe und Cu), A. M. Lahin, Zhur. Obschei Khim, *18,* 308 (1948), vgl. CA *44,* 1079b (Reduktionsmittel Zn, Al, $CuSO_4$), US-Patent 1.791.309 (Reduktionsmittel Zn + Al). Unter diesen hat die Verwendung von Eisen als Reduktionsmittel die grösste praktische Bedeutung erlangt. Jedoch hat die Verwendung von Eisen als alleiniges Reduktionsmittel grosse wirtschaftliche und ökologische Nachteile, indem mindestens 2 Mol Eisen pro Mol Anthrachinon eingesetzt werden müssen. Dies bedingt, dass z. B. pro Mol hergestelltem Benzanthron 2,0 Mol Eisensulfat oder auf 1 000 g hergestelltes Benzathron mindestens 1 320 g Eisensulfat als Abfallprodukt anfallen. Zusätzlich entstehen grosse Mengen Abfallschwefelsäure, da zur Isolierung des gebildeten Benzanthrons die Schwefelsäure auf ca. 20 % zu verdünnen ist, welche dann entweder energieaufwendig wieder zu konzentrierter Schwefelsäure regeneriert werden muss oder deren Beseitigung ein Umweltproblem darstellt.

Die Reaktion gemäss dem neuen Verfahren findet *in situ,* in einer Stufe statt.

Der Syntheseweg des erfindungsgemässen Verfahrens verläuft z. B. nach folgendem Reaktionsschema :

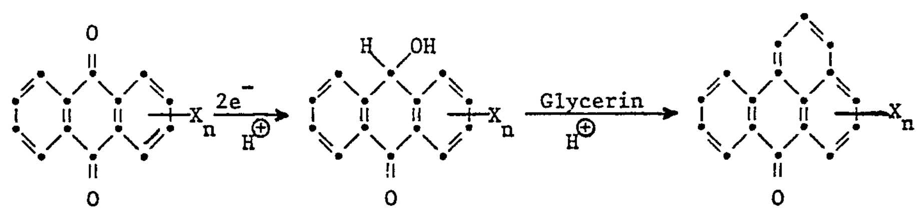


I

worin

X unabhängig voneinander OH, O-Alkyl $C_1$-$C_4$, oder Halogen und n = 0,1 oder 2 bedeutet.

Die Molverhältnisse von chinoider Verbindung zu Glyzerin können im neuen Verfahren in weiten Grenzen zwischen 1 : 1 und 1 : 10 variieren, jedoch erhält man die besten Ausbeuten bei einem vorteilhaften Molverhältnis von chinoider Verbindung zu Glyzerin von 1 : 1,5 bis 1 : 2,5. Reaktionsmedien sind vorteilhaft starke Mineralsäuren, insbesondere Schwefelsäure. Diese kann mit einer Konzentration von 60-98 %, vorzugsweise jedoch von 80-90 % eingesetzt werden. Die Verwendung organischer reaktionsinerten Säuren ist ebenfalls möglich.

Die elektrochemische Synthese läuft bei einer Temperatur zwischen 20 und 150 °C ab. Wegen der Löslichkeit, bzw. Suspendierbarkeit der chinoiden Verbindungen z. B. im schwefelsauren Elektrolyten müssen jedoch, um in technisch interessanten Konzentrationen arbeiten zu können, Betriebstemperaturen im Bereich von etwa 80 bis 120 °C gewählt werden.

Vorteilhaft arbeitet man unter Schutzgasatmosphäre, damit keine oxidative Gegenreaktion stattfindet. Dazu genügt es, wenn das Reaktionsgefäss über Reaktionsmedium-Niveau leicht, z. B. mit Stickstoff, begast wird.

Als Elektrolysenzelle kann jede Zelle mit Diaphragma gewählt werden, wobei das Diaphragma säurefest, gegen konzentrierte und verdünnte Mineralsäure, wie z. B. Schwefelsäure, Salzsäure, Fluorwasserstoff, und gegen organische Säuren wie z. B. Essigsäure, Ameisensäure, organische Sulfosäuren und andere, sein muss. Als Diaphragma eignet sich z. B. Glas oder polymere Verbindungen wie Nafion®. Die sauren Lösungen von Reaktionssubstrat und Produkt können auch organische Lösungsmittel enthalten. Dabei ist es auch möglich, dass der saure, ev. organische Lösungsmittel enthaltende Elektrolyt nicht dehydratisierend wirkt und die Synthese nur über elektrochemisch erzeugte Zwischenstufen verläuft, die auch anderweitige Reaktionen eingehen können.

Als Elektrolysezellen können auch Monozellen eingesetzt werden, die also kein Diaphragma benötigen.

Als Kathode und Anode kommen für elektroorganische Reaktionen übliche Werkstoffe, wie etwa Metalle, Metall-Legierungen, aktivierte Metalle, Metalloxid-Elektroden, Kohle-Elektroden, « Glassy-Carbon®-Elektroden » in Frage.

Das erhaltene Produkt lässt sich auf übliche Weise isolieren. Die Schwefelsäure wird z. B. auf ca. 60 % verdünnt, das ausgefallene Produkt abfiltriert und neutral gewaschen, oder das Produkt wird aus der 60 %igen Schwefelsäure mit einem handelsüblichen Lösungsmittel, extrahiert.

Zur Isolierung durch Extraktion des gebildeten Reaktionsproduktes und Phasentrennung sind höhersiedende, inerte organische Lösungsmittel, vor allem Halogenkohlenwasserstoffe, insbesondere aber Chlorbenzole, wie z. B. Monochlorbenzol, geeignet.

Die Extraktionstemperatur (Aufnahme des Produktes im organischen Lösungsmittel) beträgt 70 bis 110 °C, vorteilhaft 90 bis 100 °C.

Ein wesentlicher Vorteil, neben den schon vorher genannten, des neuen erfindungsgemässen Verfahrens ist, dass sowohl das evtl. verwendete inerte organische Lösungsmittel als auch die 60 %ige Schwefelsäure praktisch vollständig recycliert werden können. Andere Nebenprodukte bzw. Umweltbelastende Abfälle entstehen nicht. Das Produkt fällt desweiteren in einer solchen Reinheit an, dass weitere sonst übliche Reinigungsoperationen entfallen können. Es bietet somit ausserordentliche ökologische und wirtschaftliche Vorteile. Die Reaktionsprodukte sind wichtige Zwischenprodukte zur Herstellung von Farbstoffen und Pigmenten, z. B. gemäss J. Houben, W. Fischer « Anthracen und die Anthrachinone », Bd. II, Seiten 774-777 (1929) oder Venkataraman « Synthetic Dyes », Vol. II, Seiten 963-965 (1952).

Das neue Verfahren wird durch nachfolgende Beispiele beschrieben.

## Beispiel 1

In einer Elektrolysezelle aus Glas, welche aus einem durch ein Glas-Diaphragma getrennten Kathoden- (Hg-Kathode) und Anodenraum (Pt-Anode) besteht, wobei das Potential gegen eine $Hg/HgSO_4$ Bezugselektrode gemessen wird, werden 10,0 g (0,048 Mol) Anthrachinon in 110 ml Schwefelsäure 85 % suspendiert und die Lösung auf 100 °C erhitzt. Unter einer $N_2$-Schutzgasatmosphäre wird das Anthrachinon nach Stromanschluss an der Kathode zu Oxanthron reduziert, wobei gleichzeitig 9,8 g (0,1 Mol) Glyzerin zugetropft wird.

Nach Durchgang von 10 700 Coulomb pro 0,048 Mol Anthrachinon wird die Elektrolyse abgebrochen, die 85 %ige Schwefelsäure auf 60 %ige Schwefelsäure verdünnt und das ausgefallene Benzanthron abgenutscht und neutral gewaschen.

Das Rohbenzanthron wird getrocknet. Es fällt in einer solchen Reinheit an, dass es sofort in weiteren Raktionen, z. B. zur Darstellung von Violanthron oder zur Darstellung von 3-Brombenzanthron verwendet werden kann.

Ausbeute an Benzanthron mit dem Fp 167-169 °C :

9,2 g ≙ 83,25 % der Theorie.

## Beispiel 2

In einem zweiten Versuch wurde analog wie in Beispiel 1 verfahren, jedoch lag die Reaktionstemperatur bei 90 °C und der Stromdurchsatz bei 11 850 Coulomb. Es wurde 10,8 g ≙ 97,74 % d. Theorie an Benzanthron mit dem Fp 165-168 °C erhalten.

## Beispiel 3

In einer Elektrolysenzelle aus Glas, welche kein Diaphragma enthält (Monozelle), wird zwischen einer Hg-Kathode und einer Pt-Anode, wobei das Potential gegen eine $Hg/HgSO_4$ Bezugselektrode gemessen wird, 15,0 g (0,072 Mol) Anthrachinon in 250 ml Schwefelsäure 85 % suspendiert und die Lösung auf 100 °C erhitzt. Unter einer $N_2$-Schutzgasatmosphäre wird das Anthrachinon nach Stromanschluss an der Kathode zu Oxanthron reduziert, wobei gleichzeitig 11,9 g (0,13 Mol) Glyzerin zugetropft wird.

Nach Durchgang von 17 000 Coulomb wird die Elektrolyse abgebrochen, die 85 %ige Schwefelsäure auf 60 %ige Schwefelsäure verdünnt und das ausgefallene Benzanthron abgenutscht und neutral gewaschen.

Das Rohbenzanthron wird getrocknet.

Ausbeute an Rohbenzanthron mit dem Fp 158-162 °C

12,4 g ≙ 74,9 % der Theorie.

## Beispiel 4

Wie in Beispiel 1 beschrieben, werden in der gleichen Elektrolysezelle hydroxysubstituierte

Benzanthrone dargestellt aus 1-Methoxyanthrachinon (4a), 1,2-Dihydroxyanthrachinon (4b), 2-Hydroxyanthrachinon (4c), deren Ergebnisse tabellarisch aufgeführt sind.

| Verbindung | mol | Glyzerin | $H_2SO_4$ | Coulomb | T | Ausbeute | erhaltene Verbindungen |
|---|---|---|---|---|---|---|---|
| 4a | 0,05 | 0,1 mol | 150 ml 96 % | 28 000 | 90 °C | 70 % | 6-Hydroxybenzanthron 16,0 % 6-Methoxybenzanthron 79,3 % |
| 4b | 0,025 | 0,05 mol | 200 ml 85 % | 7 200 | 95 °C | 30 % | 5,6-Dihydroxybenzanthron und 70 % 1,2-Dihydroxyanthrachinon zurückgewonnen |
| 4c | 0,025 | 0,05 mol | 150 ml 85 % | 8 800 | 95 °C | 66 % | 4-Hydroxybenzanthron |

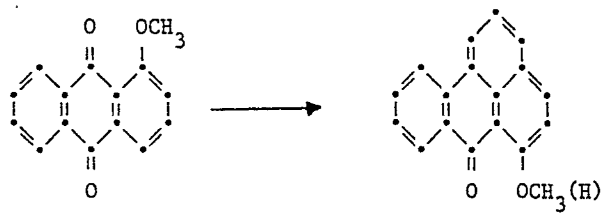

4a

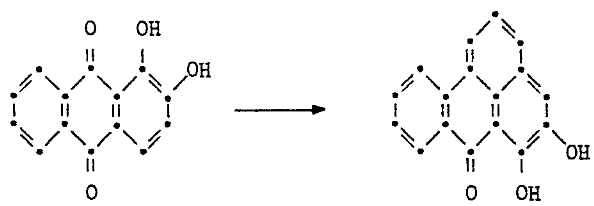

4b

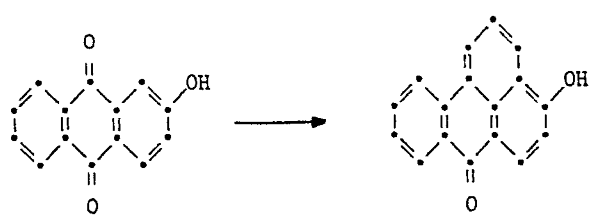

4c

Beispiel 5

Wie in Beispiel 1 beschrieben, werden in der gleichen Elektrolysezelle substituiertes Benzanthron dargestellt aus 1-Chloranthrachinon deren Ergebnisse tabellarisch aufgeführt sind.

| Verbindung | mol | Glyzerin | $H_2SO_4$ | Coulomb | T | Ausbeute | erhaltene Verbindungen |
|---|---|---|---|---|---|---|---|
| 5a | 0,15 mol | 0,5 mol | 500 ml 85 % | 60 000 | 85 °C | 29,5 % | 6-Chlorbenzanthron 86,1 % 6-Hydroxybenzanthron 13,6 % |

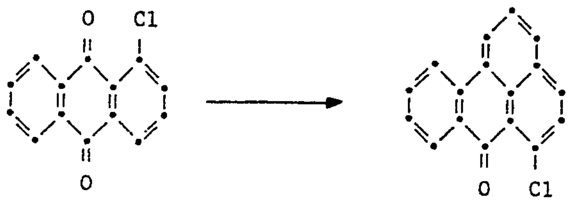

5a

## Ansprüche

1. Elektrochemisches Verfahren zur Herstellung von Benzanthron ausgehend von Anthrachinon und dessen Reaktion mit Glyzerin, dadurch gekennzeichnet, dass man Anthrachinon, das noch elektronegative Substituenten enthalten kann, in einer Elektrolysezelle in saurem Medium kathodisch reduziert und die reduzierte Semichinonform gleichzeitig mit dem Glyzerin unter Ringschluss zum entsprechenden Benzanthron reagieren lässt.

2. Verfahren gemäss Anspruch 1 mit folgendem Reaktionsschema

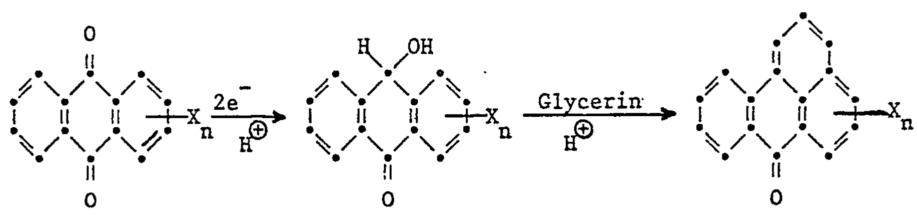


I

worin

X unabhängig voneinander OH, O-Alkyl $C_1$-$C_4$, oder Halogen und

n = 0, 1 oder 2

bedeutet.

3. Verfahren nach Anspruch 1, dadurch gekennzeichnet, dass man die Reaktion bei einer Temperatur zwischen 20 und 150 °C durchführt.

4. Verfahren nach Anspruch 1, dadurch gekennzeichnet, dass man die Reaktion bei einer Temperatur zwischen 80 und 120 °C durchführt.

5. Verfahren nach Anspruch 1, dadurch gekennzeichnet, dass man als saures Medium Mineralsäuren einsetzt.

6. Verfahren nach Anspruch 1, dadurch gekennzeichnet, dass man als saures Medium Schwefelsäure der Konz. 60 bis 98 % einsetzt.

7. Verfahren nach Anspruch 1, dadurch gekennzeichnet, dass man die chinoide Verbindung zu Glyzerin in einem Molverhältnis von 1 : 1 bis 1 : 10 einsetzt.

8. Verfahren nach Anspruch 1, dadurch gekennzeichnet, dass man Anthrachinon in einer Elektrolysezelle in 85 %iger Schwefelsäure kathodisch reduziert und gleichzeitig Glyzerin im Molverhältnis 1 : 2 zugibt, nach beendeter Reaktion die Säure auf 60 % verdünnt, das als produkt erhaltene Benzanthron neutral wäscht und trocknet.

## Claims

1. An electrochemical process for the production of benzanthrone by reaction of anthraquinone, as starting material, with glycerol, which process comprises reducing anthraquinone, which may additionally contain electronegative substituents, at the cathode in an electrolytic cell in acid medium, and simultaneously cyclising the reduced semiquinone form with glycerol to produce the corresponding benzanthrone.

2. A process according to claim 1 with the reaction scheme

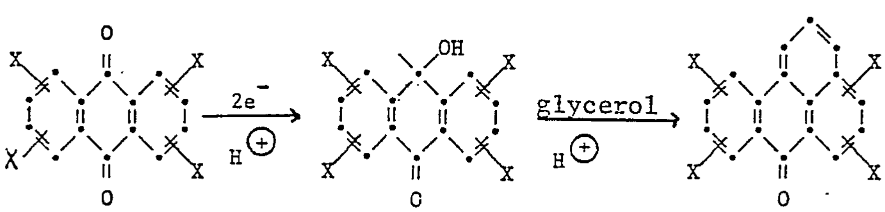


I

wherein

each X independently is OH, O-alkyl of 1 to 4 carbon atoms or halogen, and

n is 0, 1 or 2.

3. A process according to claim 1, wherein the reaction is carried out at a temperature between 20° and 150 °C.

4. A process according to claim 1, wherein the reaction is carried out at a temperature between 80° and 120 °C.

5. A process according to claim 1, wherein a mineral acid is used as acid medium.

6. A process according to claim 1, wherein sulfuric acid having a concentration of 60 to 98 % is used as acid medium.

7. A process according to claim 1, wherein the molar ratio of quinoid compound to glycerol is 1 : 1 to 1 : 10.

8. A process according to claim 1, which comprises reducing anthraquinone at the cathode in 85 % sulfuric acid in an electrolytic cell and simultaneously adding glycerol in the molar ratio of 1 : 2, diluting the acid to a concentration of 60 % after completion of the reaction, and washing the benzanthrone obtained as product until neutral and drying it.

**Revendications**

1. Procédé électrochimique pour la préparation de benzanthrone en partant de l'anthraquinone et de sa réaction avec la glycérine, caractérisé par le fait qu'on réduit par voie cathodique l'anthraquinone, qui peut contenir encore des substituants électronégatifs, en milieu acide, dans une cellule d'électrolyse, et qu'on fait réagir la forme semi-quinonique réduite simultanément avec la glycérine par cyclisation pour avoir la benzanthrone appropriée.

2. Procédé selon la revendication 1 ayant le schéma réactionnel suivant

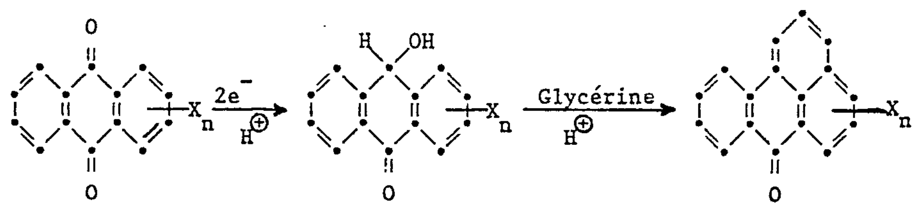


I

dans lequel

les X, indépendamment les uns des autres, sont OH, un groupe O-alkyle en $C_1$-$C_4$ ou de l'halogène, et

n = 0, 1 ou 2.

3. Procédé selon la revendication 1, caractérisé par le fait qu'on effectue la réaction à une température comprise entre 20° et 150 °C.

4. Procédé selon la revendication 1, caractérisé par le fait qu'on effectue la réaction à une température comprise entre 80° et 120 °C.

5. Procédé selon la revendication 1, caractérisé par le fait qu'on utilise comme milieu acide, des acides minéraux.

6. Procédé selon la revendication 1, caractérisé par le fait qu'on utilise comme milieu acide, de l'acide sulfurique ayant une concentration de 60 à 98 %.

7. Procédé selon la revendication 1, caractérisé par le fait qu'on utilise le composé quinoïde par rapport à la glycérine dans un rapport molaire de 1 : 1 à 1 : 10.

8. Procédé selon la revendication 1, caractérisé par le fait qu'on réduit par voie cathodique l'anthraquinone dans l'acide sulfurique à 85 %, dans une cellule d'électrolyse et qu'on ajoute simultanément la glycérine dans un rapport molaire de 1 : 2, qu'on dilue l'acide à 60 % une fois la réaction terminée qu'on lave jusqu'à neutralité et qu'on sèche la benzathrone obtenue comme produit.